(19) Europäisches Patentamt
European Patent Office
Office européen des brevets

(11) Publication number: **0 403 668 A1**

(12) **EUROPEAN PATENT APPLICATION**

(21) Application number: **89111029.8**

(51) Int. Cl.[5]: **C12Q 1/26**

(22) Date of filing: **17.06.89**

(43) Date of publication of application: **27.12.90 Bulletin 90/52**

(84) Designated Contracting States: **DE FR GB**

(71) Applicant: **ORIENTAL YEAST CO., LTD.**
**6-10, Azusawa 3-chome itabashi-ku**
**Tokyo(JP)**

(72) Inventor: **Lee, Hunmyoug Shiramine Jutaku**
**1-go, 4-8**
**Tachibana 1-chome Nishinari-ku Osaka-shi**
**Osaka-fu(JP)**

Inventor: **Nishikawa, Atsushi**
**14-10, Makiochiai 1-chome Mino-shi**
**Osaka-fu(JP)**
Inventor: **Taniguchi, Naoyuki**
**Raionzumanshon-Toyonakaueno**
**201-go, 1-38 Uenonishi 3-chome**
**Toyonaka-shi**
**Osaka-fu(JP)**

(74) Representative: **Thomsen, Dieter, Dr.rer.nat.**
**Kaiser-Ludwig-Platz 6**
**D-8000 München 2(DE)**

(54) **Method of measuring SOD activity.**

(57) This invention relates to a method of measuring the activity of superoxide dismutase ( SOD ). More particularly, it relates to a simple method of rapidly measuring the activity of SOD by the use of allopurinol as reaction terminator, whereby a large number of samples can be treated simultaneously in small quantities.

FIG. 1

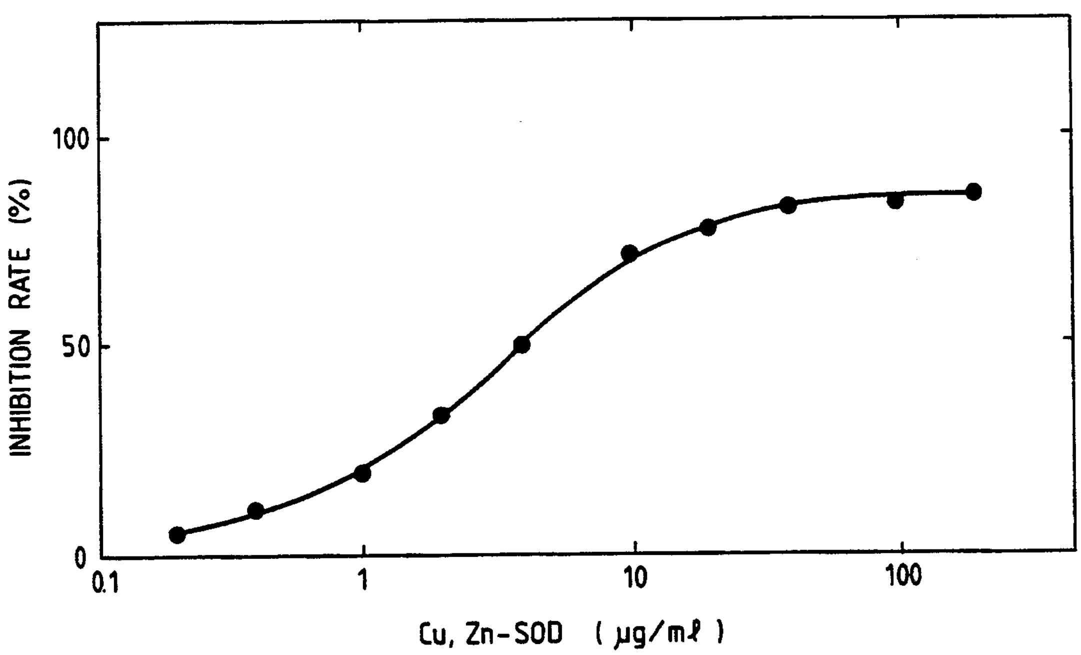


EP 0 403 668 A1

# METHOD OF MEASURING SOD ACTIVITY

This invention relates to a method of measuring the activity of superoxide dismutase ( SOD ). More particularly, it relates to a simple method of rapidly measuring the activity of SOD by the use of allopurinol as reaction terminator, whereby a large number of samples can be treated simultaneously in small quantities.

Thus, the method of this invention enables efficient diagnosis of diseases in which SOD or $O_2^-$ participates and efficient determination of purification degree in the process of SOD isolation, extraction and purification, and is therefore of great utility in the fields of medical service and biotechnology ( e.g., enzyme chemistry and biochemistry ).

## Description of the Prior Art

Superoxide dismutase ( SOD ) is a group of enzymes that decompose $O_2^-$ ( an active oxygen ) through the dismutation reaction as shown below, and may be divided into three types ( Cu,Zn-SOD, Fe-SOD and Mn-SOD ) according to the type of metal serving as active center [ Anal. Biochem., 44,276-287 ( 1971 )]:

$$2O_2^{-}\cdot + 2H^{-} \xrightarrow{SOD} H_2O_2 + O_2$$

SOD activity has been commonly measured by determining the inhibitory amount of $O_2^-$ production in the reaction system by the use of the cytochrome C method [ McCord, J.M. & Fridovich. I.: J. Biol. Chem., 244, 6049-6055 ( 1969 )], by the use of nitroblue tetrazolium ( NBT ) [ Beauchamps, C. & Fridovich, I.: Anal. Biochem., 44, 276-287 ( 1971 )], by the use of lactate dehydrogenase ( LDH ) and NADH ( Chan, P.C. & Bielski, B.H.J.: J. Biol. Chem., 249, 1317-1319 ( 1974 )], or by the use of epinephrine [ Misra, H.P. & Fridovich, I.: J. Biol. Chem., 247, 3170-3175 ( 1972 )]. In any of these methods, the change in absorbance of the reaction system is monitored over a period of one minute, and hence the measurement must be made individually for each cell tube.

The method of this invention enables simultaneous measurement of many samples in a short time by the use of allopurinol as reaction terminator, and no such technique has so far been disclosed.

## Problems to be Solved by the Invention

Measurement of SOD activity involves an inherent restriction that the reaction of SOD to dismutate $O_2^-$ must be continously monitored because the substrate $O_2^-$ is very unstable. In addition, the conventional methods mentioned above do not allow the adoption of one point assay which is capable of treating many samples simultaneously, and are hence unavoidable from the serious defect that measuring operations cannot be mechanized.

## Brief Description of the Drawings

Figure 1 is a standard curve when inhibition rate is measured by the use of Cu,Zn-SOD purified from human erythrocytes.

Figure 2 is a graph illustrating the stability of developed color.

Figure 3 is a graph illustrating the relationship between allopurinol concentration and inhibition rate.

Figure 4 is the absorption spectrum after termination of the reaction.

## Means to Solve the Problems

The object of this invention is to provide a new method free from the above-mentioned problems associated with conventional methods. The method is capable of correctly and simultaneously analyzing a

large number of samples in a short time with a minimum of measurement error despite the use of a very unstable substrate, $O_2^-$, thus enabling automatic analysis.

This invention, which satisfies very severe requirements as described above, is a result of extensive and thoroughgoing studies.

In practicing this invention, it is necessary that the substrate for SOD, $O_2^-$, be stably supplied. As the reaction to generate $O_2^-$, may be used photoreduction of flavin, the potassium peroxide method, the electrochemical method, autoxidation of pyrogallol, and others. Of these, the use of xanthine-XOD system, in which xanthine is catalyzed with xanthine oxidase ( XOD ), is the most preferred.

As the method of measuring the amount of $O_2^-$ thus formed, may be mentioned the cytochrome C method, the nitroblue tetrazolium ( NBT ) method, reduction of tetranitromethane, oxidation of 2-ethyl-1-hydroxy-2,5,5-trimethyl-3-oxazoline, the nitrous acid method, and others. Of these, the NBT method is best suited for termination of the reaction with allopurinol ( an essential point of the method of this invention ) and also fits the xanthine-XOD system described above.

Since the reaction proceeds with the passage of time, SOD activity cannot be measured without continuous monitoring of each cell tube; hence, it is impossible to treat a large number of samples simultaneously. Under the circumstances, we hit upon the idea of treating a large number of samples simultaneously by terminating the reaction after a definite time, and made extensive studies on the selection of a suitable reaction terminator, stability of developed color, synchronous reproducibility, and correlation data. As a result, it was discovered that very satisfactory results can be obtained if allopurinol, which is an inhibitor against XOD, is used as the reaction terminator. This invention was accomplished on the basis of this finding.

The principle of SOD activity measurement by the method of this invention is as shown below.

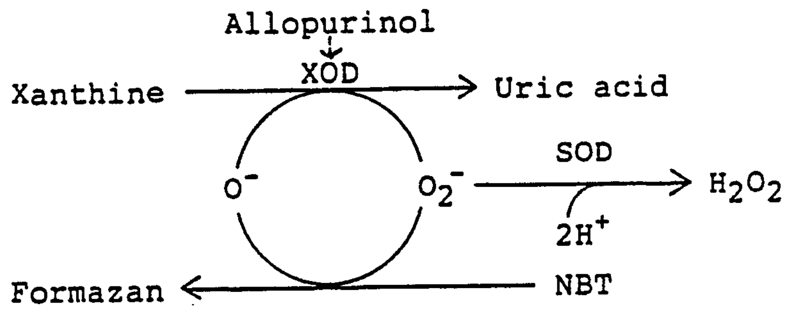


As is apparent from the above scheme, $O_2^-$ formed in the Xanthine-XOD system changes NBT into formazan, thus developing a blue color. In this case, if SOD is present in the test sample, it consumes $O_2^-$ and the result is a lowered degree of color development. This reaction is allowed to proceed for a definite time ( which may vary with the amounts of sample and substrate, but is in the range of 1 to 15 minutes; normally, five minutes ), and allopurinol is then added to stop the formation of $O_2^-$, thereby terminating the overall reaction.

SOD activity is determined by measuring the blue color of formed formazan with a spectrophotometer ( measuring absorbance at 560 nm, for example ), followed by calculation with the value in the absence of SOD taken as 0% inhibition.

The method of this invention may, of course, be carried out in the same manner as in conventional methods ( using test tubes or cells, and making measurement individually for each sample ), but it is also possible to treat many samples simultaneously by the use of a microtiter plate provided with a number of wells. SOD activity measurement can thus be mechanized for the first time, enabling its automatic analysis.

As the microtiter plate, may be used a plastic plate with a number of wells bored thereon or a tray with a plurality of sample cells or inspection holes made thereon. Also may be employed commercial products used for immune reactions, immunoassay and enzyme analysis, such as microtiter trays, microtrays, microplates, depression trays and micro-CFT trays.

The method of this invention is novel in that the reaction is terminated by the use of a terminator, and allopurinol must be used as the terminator to achieve the object of this invention.

Allopurinol is 1H-pyrazolo[3,4-d]pyrimidin-4-ol having the following structural fromula:

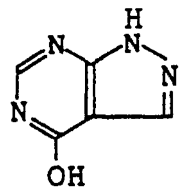

This particular compound, when used to terminate the formation of $O_2^-$ in the reaction system, causes no effect upon the other reactions involved, thereby stabilizing the developed color and ensuring correct measurement of SOD activity. This fact is a novel technical finding of great value which was heretofore unknown at all.

The method of this invention assures correct measurement even with very small amounts of samples used. In addition, a large number of samples can be treated simultaneously if a plastic plate provided with many wells ( e.g., a microtiter plate ) is used. Furthermore, activities of Cu,Zn-SOD and of Mn-SOD can be measured separately by addition of NaCN or KCN solution, as shown in Table 1.

The following examples describe the procedures for measuring SOD activity in which a microtiter plate made of polystyrene is used.

Example 1

The reagents enumerated below were used in this experiment.

(1) Solution of ammonium sulfate

2M solution of ammonium sulfate containing 1mM EDTA-Na.

(2) XOD solution

A solution prepared by adding 10 µl of XOD stock solution ( 100U/5ml ) to 1 ml of the above ammonium sulfate solution .

(3) Assay mixture solution

A solution containing $4 \times 10^{-5}$M NBT, $1.6 \times 10^{-4}$M xanthine, $1.6 \times 10^{-4}$M EDTA-2Na and 80mM $Na_2CO_3$, which is prepared by dissolving 30.9 mg NBT, 24.34 mg xanthine, 59.56 mg EDTA-2Na and 8.48 g $Na_2CO_3$ in about 800 ml distilled water, and adjusting the pH to 10.2 by addition of 1N-HCl.

(4) Allopurinol solution ( 0.84mM )

A solution prepared by dissolving 114.3 mg allopurinol ( M.W.: 136.11 ) in 1000 ml distilled water.

(5) NaCN or KCN solution ( 12mM )

Measurement of SOD activity was made in each of the wells of the microtiter plate by using the above reagents according to the procedure shown in Table 1.

To each of the wells, were added 20 µl of a test sample ( $H_2O$ for the blank ), 20 µl of $H_2O$ ( the KCN or NaCN solution for measurement of Mn-SOD activity ), and 20 µl of the XOD solution ( the ammonium sulfate solution for the blank ).

The assay mixture solution ( 100 µl ) was then added to each well, and the reaction was allowed to proceed exactly for five minutes and terminated by adding the allopurinol solution ( 50 µl each ).

Absorbance at 560 nm was measured using a spectrophotometer ( immunoreader ), and $E_S$, $E_B$ and $E_{SB}$ values were recorded, with the blank value ( $E_{BB}$ ) used as zero-point correction.

SOD activity was then calculated from the following equation:

$$\text{SOD Activity ( Inhibition rate \% )} = \left\{1 - \left(\frac{E_S - E_{SB}}{E_B}\right)\right\} \times 100$$

Table 1

| Measurement of Cu,Zn-SOD and Mn-SOD activities on a microtiter plate | | | | |
|---|---|---|---|---|
| | Run Proper | | Blank Test | |
| | Sample (S) | Blank (B) | Sample (SB) | Blank (BB) |
| Test sample | 20 µl | - | 20 µl | - |
| $H_2O$ | - | 20 µl | - | 20 µl |
| *$H_2O$, KCN or NaCN soln. | 20 µl | 20 µl | 20 µl | 20 µl |
| XOD soln. | 20 µl | 20 µl | - | - |
| $(NH_4)_2SO_4$ soln. | - | - | 20 µl | 20 µl |

* $H_2O$ for measurement of Cu,Zn-SOD activity; KCN or NACN soln. for measurement of Mn-SOD activity; when KCN is added for measurement of Mn-SOD activity, the mixture is incubated in this step for five minutes to deactiv te Cu,Zn-SOD.

| | | | | |
|---|---|---|---|---|
| Assay mixture | 100 µl | 100 µl | 100 µl | 100 µl |

Reaction is continued exactly for five minutes.

| | | | | |
|---|---|---|---|---|
| Allopurinol soln. | 50 µl | 50 µl | 50 µl | 50 µl |
| Measurement ( $A_{560nm}$ ) | $E_S$ | $E_B$ | $E_{SB}$ | $E_{BB}$ |

$E_S$, $E_B$ and $E_{SB}$ values were recorded by using an immunoreader ( with $E_{BB}$ used as blank value ).

Cu,Zn-SOD derived from human erythrocytes ( product of Ube Industries, Ltd. ) was used as the test sample at different concentrations, and measurement was made according to the procedure described above, giving the result shown in Figure 1.

As can be seen from the figure, inhibition rate increases in proportion to SOD concentration within the range from 0.5 to 20 µg/ml. The figure also indicates that, at SOD concentrations higher than 20 µ g/ml ( with inhibition rates higher than 75% ), the test sample should be further diluted prior to measurement in order to obtain more accurate results.

Example 2

Samples of different activity values ( SOD obtained from human erythrocytes by partial purification ) were each tested repeatedly in the same manner as in Example 1, and synchronous reproducibility was calculated for each sample from the absorbance data thus obtained. It is apparent from the result summarized in Table 2 that, although Sample D ( inhibition rate: 72% ) showed a slightly large coefficient of variation ( C.V.: 7.1% ), the other samples ( Sample A, inhibition rate: 18%; Sample B, inhibition rate: 52%; Sample C, inhibition rate: 58% ) showed satisfactorily low C.V. values ( 1.8%, 4.0% and 4.2%, respectively ).

Table 2

| | Control | Sample A | Sample B | Sample C | Sample D |
|---|---|---|---|---|---|
| n | 8 | 16 | 16 | 16 | 16 |
| mean ($A_{560}$) | 0.186 | 0.152 | 0.089 | 0.079 | 0.052 |
| S.D. | 0.0021 | 0.0027 | 0.0036 | 0.0033 | 0.0037 |
| C.V. (%) | 1.1 | 1.8 | 4.0 | 4.2 | 7.1 |

As can be seen from the above result, the method of this invention shows high synchronous reproducibility.

Example 3

A test sample ( product obtained from human erythrocytes by partial purification ) was treated in the same manner as in Example 1, and the stability of developed color after standing at room temperature was examined. As is apparent from the result shown in Figure 2, a slight increase in absorbance was observed after termination of the reaction both with the test sample and in the blank test, but this change, if converted to that of SOD activity ( inhibition rate % ), was negligibly small, indicating the stability of developed color sufficiently high for practical use.

As may be apparent from the foregoing, because of such outstandingly high stability of developed color, absorbance measurement can be made at any time and requires no skilled operator. The method of this invention is thus suited for treatment of many samples and for mechanization.

Studies were also made on the optimum concentration of allopurinol to terminate the reaction and on the absorption spectrum of the reaction system after termination. The results obtained are shown in Figures 3 and 4.

The minimum concentration of allopurinol that ensures termination of the reaction was found to be 50 μM, and hence the suitable concentration was set as 200 μM. Absorption spectrum measured by Shimadzu's Model UV-160 Spectrophotometer showed an absorption peak near at 560 nm.

Effects Achieved by the Invention

The method of this invention makes it possible to treat a large number of samples simultaneously. It may be effectively used for activity measurement of chromatographic fractions in the process of SOD purification and for many other purposes, and is hence of great utility.

This method may also be used extensively for the diagnosis of various diseases in which $O_2^-$ or active oxygen derived therefrom participates, such as inflammations, immune diseases, cancers, lipid oxidation abnormalities, and radiation hazards.

## Claims

A method measuring the activity of superoxide dismutase by the use of allopurinol as reaction terminator.

FIG. 1

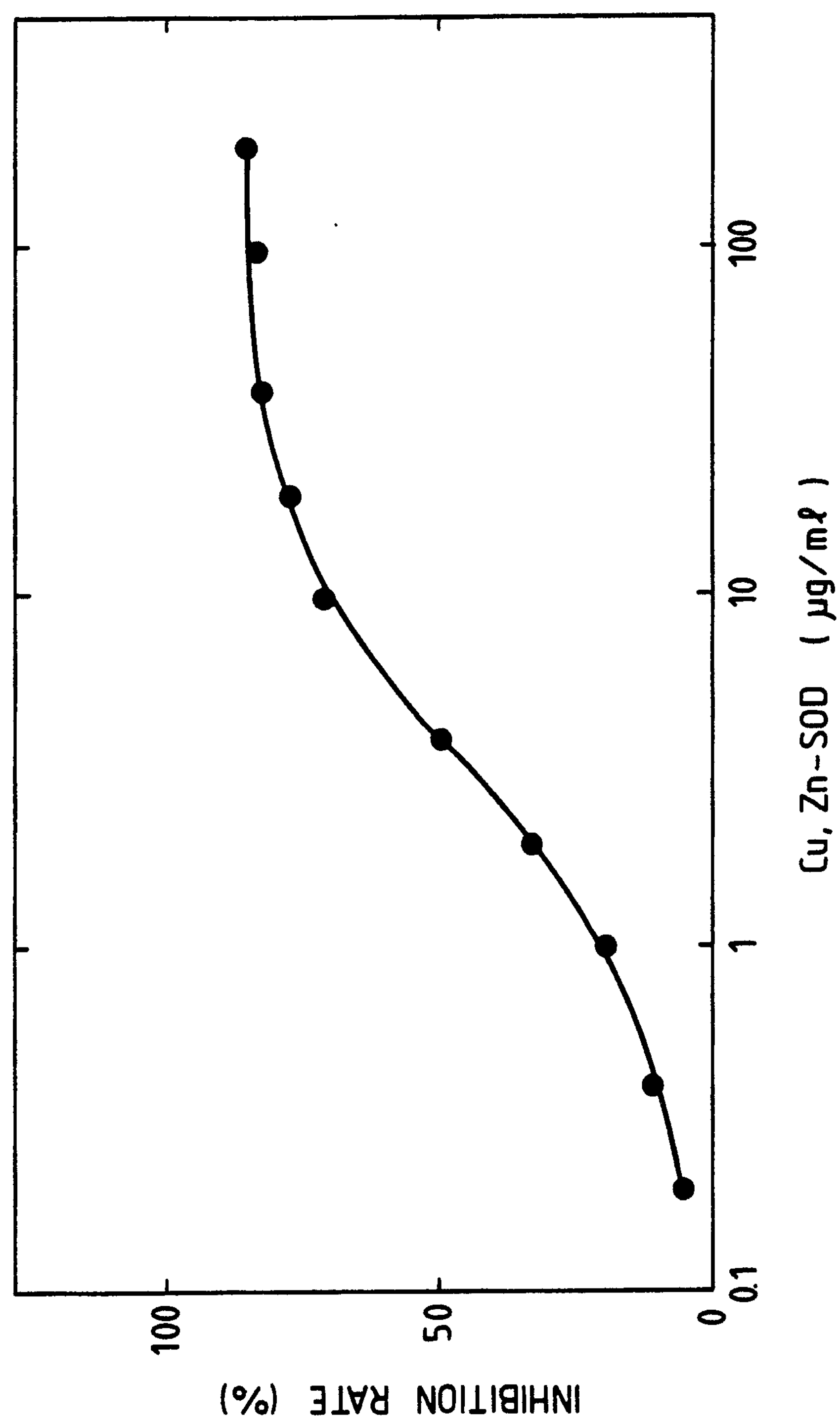

FIG. 2

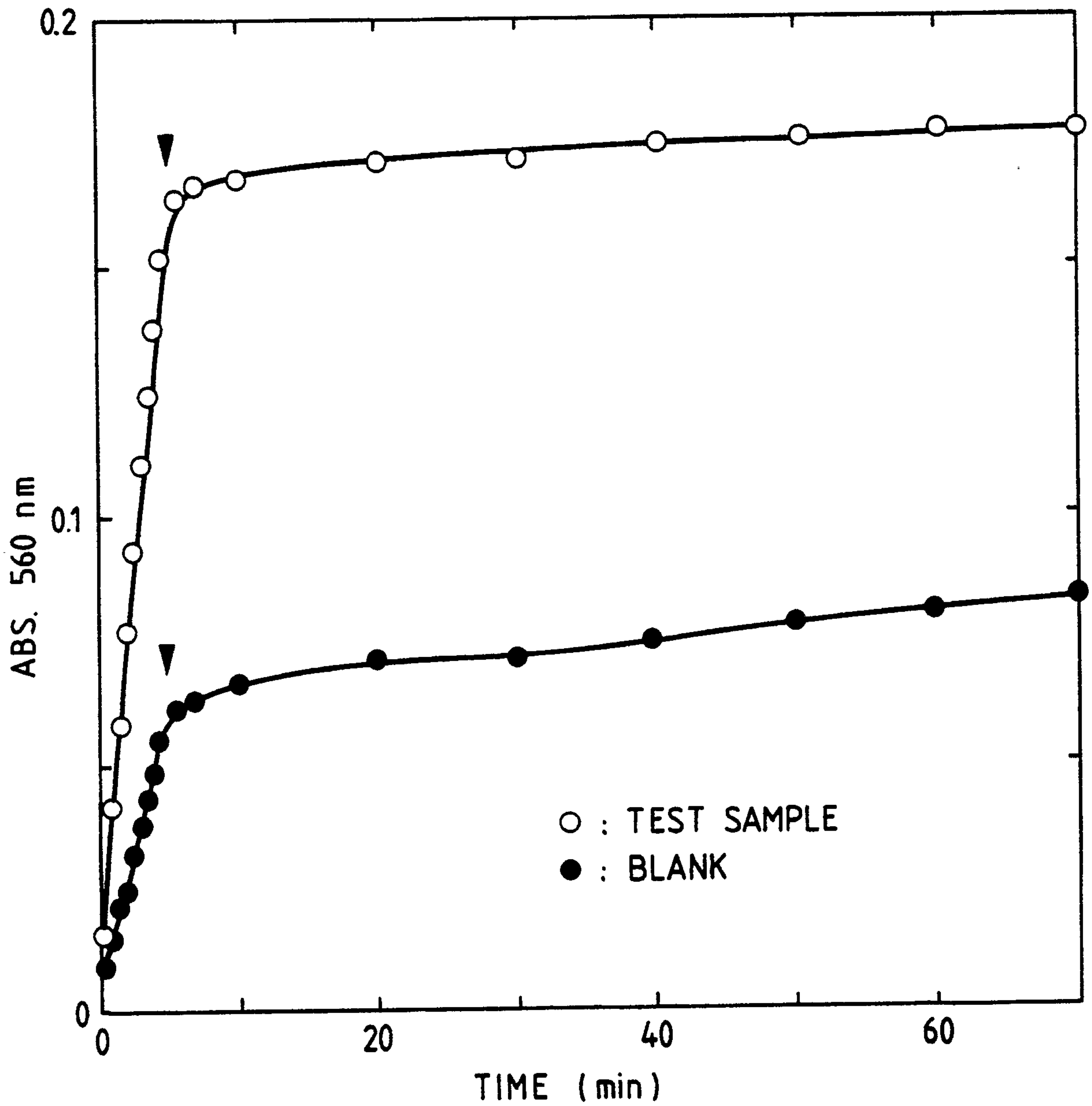

FIG. 3

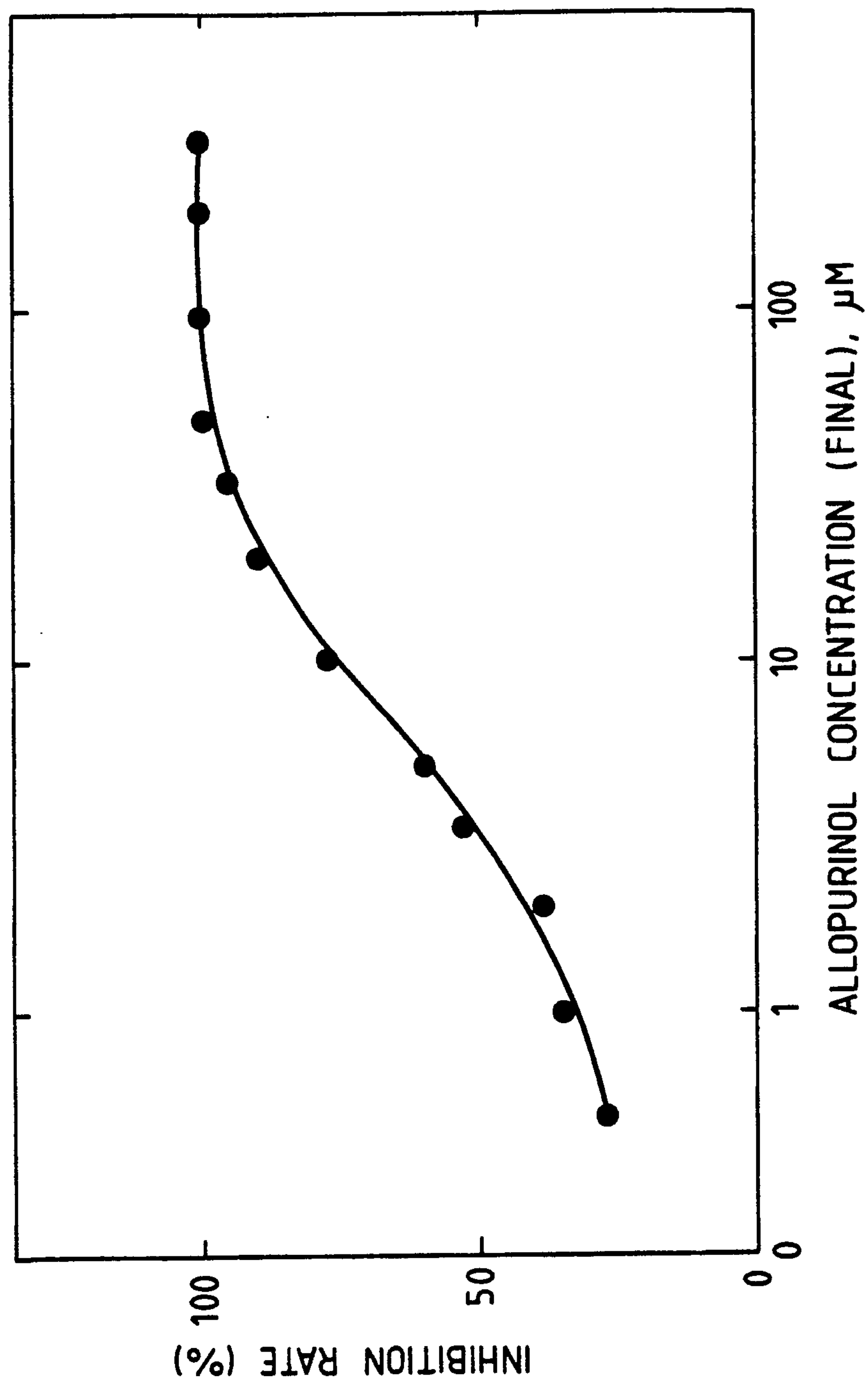
INHIBITION RATE (%)
100
50
0
0
1
10
100
ALLOPURINOL CONCENTRATION (FINAL), μM

FIG. 4

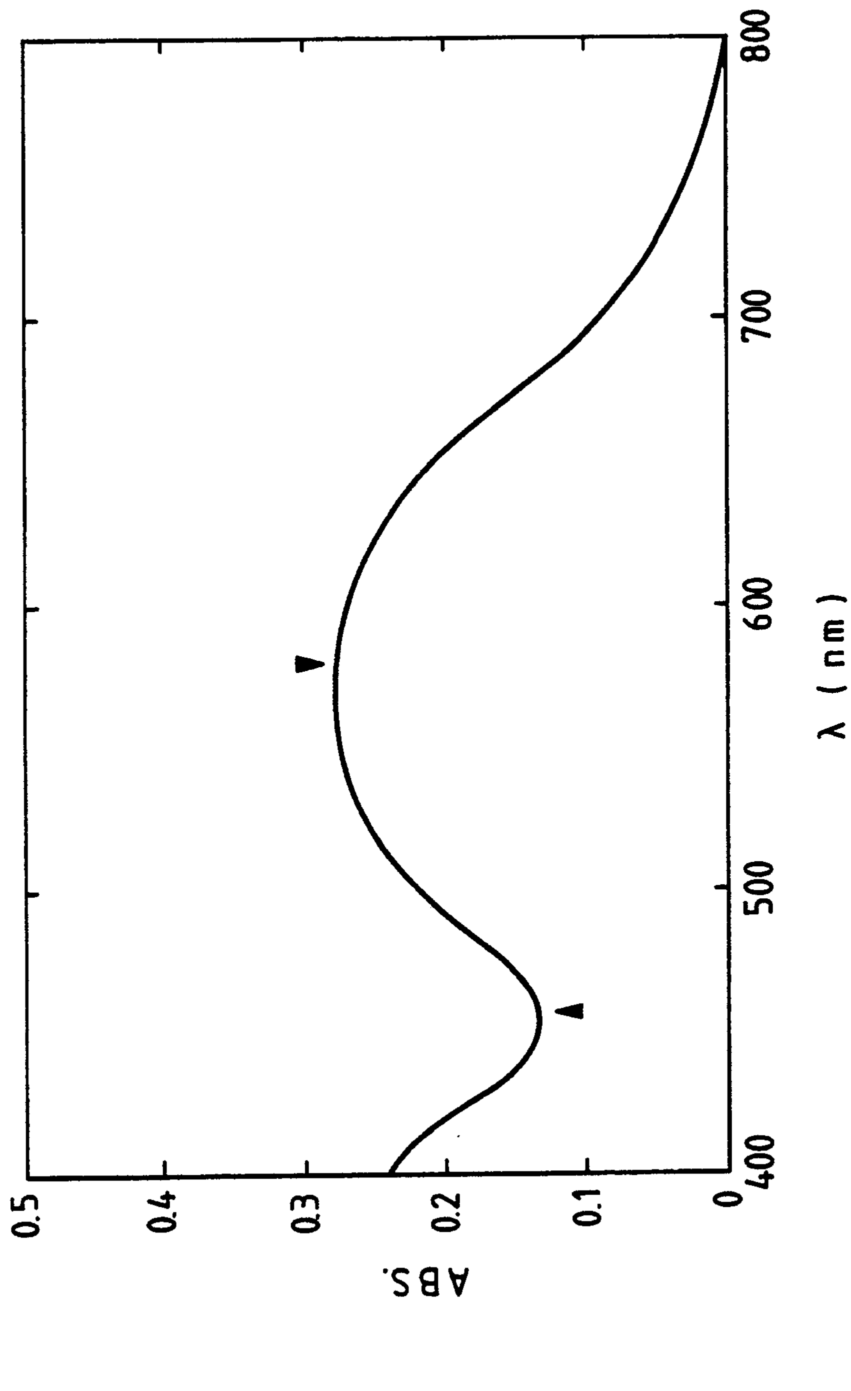

European Patent Office

# EUROPEAN SEARCH REPORT

Application Number

EP 89 11 1029

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | CLINICAL CHEMISTRY. vol. 34, no. 3, March 1988, WINSTON US pages 497 - 500; Y.Sun et al.: "A simple method for clinical assay of superoxide dismutase" * the whole document * --- | 1 | C12Q1/26 |
| Y | US-A-4238566 (J.P.ZIKAKIS) * column 9, lines 22 - 29 * --- | 1 | |
| D,A | ANALYTICAL BIOCHEMISTRY vol. 44, 1971, NEW YORK US pages 276 - 287; C.Beauchamp et al.: "Superoxide dismutase: Improved assays and an assay applicable to acrylamide gels" * the whole document * --- | 1 | |
| A | EP-A-193204 (WAKO PURE CHEMICAL INDUSTRIES LTD.) * column 6, line 59 - column 8, line 4 * --- | 1 | TECHNICAL FIELDS SEARCHED (Int. Cl.5 ) |
| E | PATENT ABSTRACTS OF JAPAN vol. 13, no. 423 (C-638)(3771) 20 September 1989, & JP-A-1 160500 (ORIENTAL YEAST CO. LTD.) 23 June 1989, * the whole document * ----- | 1 | C12Q<br>C12N |

The present search report has been drawn up for all claims

| Place of search | Date of completion of the search | Examiner |
|---|---|---|
| BERLIN | 05 FEBRUARY 1990 | DE KOK A.J. |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)